# EUROPEAN PATENT SPECIFICATION

(11) **EP 1 321 148 B1**
(45) Date of publication and mention of the grant of the patent: **24.05.2006**
(21) Application number: 02257948.6
(22) Date of filing: 19.11.2002
(51) Int. Cl.: A23B 7/154

(54) **Use of cyclotetrasaccharide for augmenting "active-oxygen-eliminating-activity"**
Verwendung von Cyclotetrasaccharide zur Erhöhung der "Aktiven-Sauerstoff-Eliminierenden-Aktivität"
Utilisation de cyclotetrasaccharides pour augmenter l'activité d'élimination de l'oxygène actif

(30) Priority: 20.11.2001 JP 2001355273
(43) Date of publication of application: 25.06.2003
(73) Proprietor: KABUSHIKI KAISHA HAYASHIBARA SEIBUTSU KAGAKU KENKYUJO, Okayama-shi, Okayama 700-0907 (JP)
(72) Inventor: Oku, Kazuyuki, Kabushiki Kaisha Hayashibara, Okayama-shi, Okayama 700-0907 (JP); Kubota, Michio, Kabushiki Kaisha Hayashibara, Okayama-shi, Okayama 700-0907 (JP); Fukuda, Shigeharu, Kabushiki Kaisha Hayashibara, Okayama-shi, Okayama 700-0907 (JP); Miyake, Toshio, Kabushiki Kaisha Hayashibara, Okayama-shi, Okayama 700-0907 (JP)
(74) Representative: Daniels, Jeffrey Nicholas

(56) References cited:
- EP-A- 0 868 916
- EP-A- 1 229 112
- EP-A- 1 284 286
- COTE G L ET AL: "ENZYMICALLY PRODUCED CYCLIC ALPHA-1,3-LINKED AND ALPHA-1,6-LINKED OLIGOSACCHARIDES OF D-GLUCOSE" EUROPEAN JOURNAL OF BIOCHEMISTRY, BERLIN, DE, vol. 226, 1994, pages 641-648, XP002945423 ISSN: 0014-2956
- BRADBROOK G M ET AL: "X-ray structure determination and modeling of the cyclic tetrasaccharide cyclo-{->6)-alpha-d-Glcp-(1->3)-alpha-d-Gl cp-(1->6)-a lpha-d-Glcp-(1->3)-alpha-d-Glcp-(1- >}" CARBOHYDRATE RESEARCH, ELSEVIER SCIENTIFIC PUBLISHING COMPANY. AMSTERDAM, NL, vol. 329, no. 3, 17 November 2000 (2000-11-17), pages 655-665, XP004221215 ISSN: 0008-6215
- BIELY P ET AL: "Enzymic alpha-galactosylation of a cyclic glucotetrasaccharide derived from alternan" CARBOHYDRATE RESEARCH, ELSEVIER SCIENTIFIC PUBLISHING COMPANY. AMSTERDAM, NL, vol. 332, no. 3, 4 June 2001 (2001-06-04), pages 299-303, XP004249620 ISSN: 0008-6215

## Description

### Background of the Invention

### Field of the Invention

The present invention relates to the use of an agent which inhibits reduction of active oxygen eliminating activity, and a method for inhibiting reduction of active oxygen eliminating activity. More particularly, the present invention relates to the use of an agent for inhibiting reduction of active oxygen eliminating activity where the agent comprises cyclo{ → 6}- α -D-glucopyranosyl-(1 → 3)- α -D-glucopyranosyl-(1 → 6)- α -D-glucopyranosyl-(1 → 3)- α -D-glucopyranosyl-(1 → ), hereinafter abbreviated as "cyclotetrasaccharide", represented by Chemical Formula 1 as an effective ingredient, a method for inhibiting the reduction of plant active oxygen eliminating activity characterized in that it comprises the steps of incorporating either cyclotetrasaccharide or the inhibitory agent in the presence of an aqueous medium.

### Description of the Prior Art

It is well known that edible plant substances such as vegetables, mushrooms, and seaweeds are important for living bodies as sources of functional ingredients such as vitamins, minerals, and edible fibers, and are essential food materials for the growth of living bodies. Recently, interest has been focused on the active oxygen eliminating activity of these edible plants with respect to the maintenance and promotion of health, the prevention of aging and geriatric disease, and the inhibition of carcinogenesis. Mechanisms for the causes of aging of living bodies and related diseases thereof such as cancers, arteriosclerosis, liver cirrhosis, myocardial infarction, cerebral apoplexy, cataracts, Parkinson's disease, rheumatism, and Alzheimer's dementia are still unknown in many aspects. However, there are some consideration that these incurable diseases may relate to active oxygen molecules such as superoxide which is an oxygen molecule having an unpaired electron and relatively high reactivity, and derivatives thereof including hydroxyradical and hydrogen peroxide. It is believed that these molecules oxidize intracellular target molecules such as membrane lipids, proteins, and DNAs to induce oxygen-related defects and cause aging of living bodies and related diseases thereof.

Enzymes capable of eliminating active oxygen such as superoxide dismutase (EC 1.15.1.1) and catalase (EC 1.11.1.6), and antioxidants such as L-ascorbic acid and α-tocopherol exist in living cells, so that the concentration of intracellular active oxygen is generally kept at a remarkably low level. However, irradiation from a relatively-large amount of ultraviolet rays, radiations, and magnetic waves, excessive physical exercise, great mental stress, and aging form active oxygen in an amount that cannot be eliminated by the active oxygen eliminating activity of living bodies. This results in an accumulation of compounds oxidized by the excessive amount of active oxygen which causes the aforesaid oxygen-related defects. To improve the problem, for example, Japanese Patent Kokai Nos. 168,435/93 and 143,466/96 proposed methods which comprise supplementing living bodies with active oxygen eliminating activity to maintain and promote health of living bodies by using a relatively-high active oxygen eliminating activity of edible plants. It was confirmed that, even if such edible plants were used, processing techniques such as squeeze, extraction, heating, and drying, or additional storage conditions for the edible plants might lower the inherent active oxygen eliminating activity of the plants or even extinguish this activity completely. It is desired to establish a novel method for inhibiting the reduction of active oxygen eliminating activity without causing serious side effects in living bodies.

### Summary of the Invention

The objects of the present invention is to provide a novel method for inhibiting the reduction of active oxygen eliminating activity.

The object of the present invention is to provide a method for inhibiting the reduction of plant active oxygen eliminating activity which is characterized in that it comprises a step of incorporating either cyclotetrasaccharide or the inhibitory agent into plant substances with active oxygen eliminating activity.

To attain the above object, the present inventors studied the use of saccharides and continued studying. They examined influence of saccharides on the reduction inhibitory effect for active oxygen eliminating activity of plant substances such as edible plant substances and plant antioxidants by incorporating saccharides therein. As a result, the present inventors found that cyclotetrasaccharide represented by Chemical Formula 1 exerts a more effective activity than other saccharides and strongly inhibits the reduction of plant active oxygen eliminating activity, and thus accomplished the present invention.

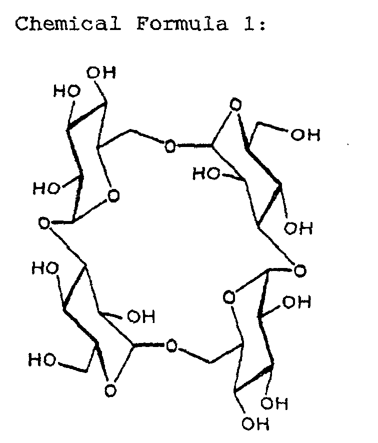

### Detailed Description of the Invention

A cyclotetrasaccharide represented by Chemical Formula 1 is a non-reducing saccharide having a cyclic structure of cyclo{→ 6}- α -D-glucopyranosyl-(1→3) - α -D-glucopyranosyl-(1 → 6) - α -D-glucopyranosyl-(1 → 3)- α -D-glucopyranosyl-(1 → ) composed of glucose molecules via the α-1,3 and α-1,6 bonds as disclosed in *Carbohydrate Research,* Vol. 329, pp. 655-665, 2000, by Gail M. Bradbrook. The saccharide can be obtained from starch by an enzyme saccharifying method which utilizes α-isomaltosylglucosaccharide-forming enzyme and α -isomaltosyl-transferring enzyme in accordance with the methods as disclosed in International Patent Application Nos. PCT/JP01/04276 and PCT/JP01/06412 by the same applicant as the present invention. Any form of cyclotetrasaccharide such as a syrup, crystalline powder of massecuite, crystalline hydrate, crystalline anhydride, or amorphous solid can be arbitrarily used. The cyclotetrasaccharide content in the present reduction inhibitory agent for active oxygen eliminating activity can be selected from those which exert a desired reduction inhibitory activity on the active oxygen eliminating activity of edible plant substance, usually, at least about 10 % (w/w) (in the present specification, "%" means % (w/w) unless specified otherwise) on a dry solid basis (d.s.b.), preferably at least about 20 %, d.s.b., and more preferably at least about 50 %, d.s.b. Cyclotetrasaccharide can be preferably incorporated into compositions containing edible plant substances and/or plant antioxidants in an amount of at least about one percent, preferably at least about five percents, and more preferably at least about 20 %, d.s.b. Generally, when the amount of cyclotetrasaccharide is less than about one percent, d.s.b., the reduction inhibitory activity for active oxygen eliminating activity may be rather insufficient. The term "on a dry solid basis (d.s.b.)" in the present invention means "under an ideal dried condition with no moisture, the weight of the composition comprising edible plant substances and/or plant antioxidants of the present invention". For example, d.s.b. can be calculated by substantially measuring a water weight of a composition, which cannot be removed in a conventional drying step, in a method such as the Karl-Fischer's method and subtracting the measured water weight from the dried weight of the composition.

The term "incorporating" in the present invention means "coexisting". In the case of incorporating the present reduction inhibitory agent or cyclotetrasaccharide as an effective ingredient into plant substances with active oxygen eliminating activity, any conventional methods can be used as long as the agent exerts a reduction inhibitory activity for plant active oxygen eliminating activity. The present agent can be preferably incorporated into plant edible substances and/or plant antioxidants by allowing cyclotetrasaccharide to contact with the substances and/or the antioxidants in the presence of an aqueous medium as homogeneously as possible. For example, in the case of using edible plant substances, etc., in a juicy form such as a liquid or suspension form, cyclotetrasaccharide in a solid form such as a powder and crystal; or a syrup form can be incorporated into plant edible substances as homogeneously as possible by mixing with and dissolving in the substances. While in the case of using edible plant substances and plant antioxidants in a solid form, they are treated with an appropriate volume of an aqueous medium to give a juicy form such as a liquid or suspension, and are incorporated with cyclotetrasaccharide by:
(i) the above method; or
(ii) providing a syrup of cyclotetrasaccharide prepared by using an appropriate volume of an aqueous medium, and dispersing, dissolving, or suspending the edible plant substances and the antioxidants in the syrup to allow to contact them with cyclotetrasaccharide as homogeneously as possible to incorporate cyclotetrasaccharide into the substances and the antioxidants.
The aqueous medium is usually water, and occasionally an aqueous solution comprising materials such as acids, bases, minerals, saccharides, alcohols, amino acids, and proteins, for example, sea water, ocean deep water, milk, serum, and soup, in an amount which does not affect the effect of the present invention. In the case of using edible plant substances such as disrupted raw plant tissues, cyclotetrasaccharide can be incorporated into the edible plant substances either by sprinkling cyclotetrasaccharide in a solid form over the substances and mixing them to melt and incorporate cyclotetrasaccharide into the substances, or by soaking the edible plant substances in cyclotetrasaccharide in a syrup form to incorporate cyclotetrasaccharide into the plant organs as homogeneously as possible. Conditions such as temperatures and times in incorporating cyclotetrasaccharide by contacting it with edible plant substances can be appropriately selected, if necessarily.

The plant substances with active oxygen eliminating activity which can be used in the present invention mean edible plants *per se;* disrupted, minced, pulverized, dried, pickled, and extracted products of edible parts of the edible plants; and/or plant antioxidants. Examples of the edible plants arbitrarily used in the present invention are edible root crops such as carrots, lotus roots, onions, burdocks, Japanese radishes, taros, yams, sweet potatoes, and potatoes; leafy vegetables such as lettuces, chicories, Chinese cabbages, cabbages, kales, moreheiya or Corchorus olitorius, *Angelica keiskei*, spinach, Malabar nightshades, *Brassica campestris, Brassica rapa, Chrysanthemum aoronirium,* ging-geng-cai or pak-choi, and turnips; fruiting vegetables such as gumbos, cauliflowers, broccolis, eggplants, tomatoes, cucumbers, pumpkins, zucchini, green peppers, field peas, and kidney beans; vegetables such as alfalfa, bean sprouts including those of soy beans and mung beans; mushrooms such as Chinese mushrooms or *Lentinus edodes,* winter mushrooms or *Celtis sinensis,* and shimeji mushrooms or *Lyophyllum shimeji;* seaweeds such as *Hijikia fusiformis,* Undaria pinnatifida, and tangs; citrus fruits such as lemons, citrons, and *"sudachi"* (a kind of citrus fruit), shaddocks, and kumquats; fruits such as bananas, pineapples, kiwifruits, strawberries, hawthorns, blueberries, grapes, peaches, apples, pears, and chestnuts; herbs such as garlic, gingers, wasabis (a Japanese horseradish), mustards, parsleys, Chinese parsleys, beefsteak plants, leeks, Welsh onions, celeries, dropworts, cressons, Guinea peppers, Japanese peppers, peppers, rosemaries, and mints; medicinal herbs such as mugworts, common plantains, *"dokudami"* or bad-smelling perennial plants of the family *Sauruaceae, Cassia obtusifolia,* Japanese green gentians, aloes, licorices, turmerics, Japanese indigo plants, Pfaffia, loquat leaves, field horsetails, pine needles, bamboo leaves, umes or Japanese apricots, tea leaves, barley leaves, wheat leaves, oat leaves, rye leaves, buckwheat leaves, ginkgo leaves, Chinese gutta percha leaves, oobanasarusuberi (a plant of the family *Lythrum), Aspalathus linearis,* and *Gymnema sylvestre*; and others including edible parts of nuts, seeds, and germs such as adlays, buckwheat's, sesames, rices, wheats, corns, broad beans, soybeans, peanuts, walnuts, pine seeds, other seeds and germs, pistachio nuts, almonds, cashew nuts, and macadamia nuts. Pickled products of the edible plants, for example, salted, *"nukazuke"* (seasoned in bran paste), *"koujizuke"* (seasoned in malt), *"kasuzuke"* (seasoned in lees), *"tamarizuke"* (seasoned products in brewed soy sauce), seasoned with vinegar, and Korean pickled vegetables such as the aforesaid root crops, leafy vegetables, and fruiting vegetables , can be arbitrarily used.

One or more of the following relatively-low molecular weight plant antioxidants can be arbitrarily selected and used as the present plant antioxidants: plant enzymes having an active oxygen eliminating activity such as superoxide dismutase, catalase, and peroxidase; plant pigments such as rutin, α-glucosyl rutin, hesperidin, α-glucosyl hesperidin, naringin, α-glucosyl naringin, chlorophyll, carotenoid, and anthocyanin; plant polyphenols such as gallic acid, catechin, *α*-glucosyl catechin, tannic acid, enzogenol (pine coat extracts), and grape seed extracts; and plant vitamins such as α-tocopherol, L-ascorbic acid, and riboflavin. In the present invention, the relatively-low molecular weight plant antioxidants can be contained in the edible plants or added to the contents during processing of the edible parts of edible plants, if necessarily. The plant antioxidants, which exist separately from the edible plants, can be used in the present invention. These plant antioxidants should not be restricted to those which are extracted from plants, and include those which are artificially synthesized and produced.

More concretely, the processes for producing compositions according to the present invention, where the reduction of the active oxygen eliminating activity is satisfactorily inhibited, are as follows: Edible parts of edible plants are, for example,
(i) disrupted or cut by mixers, juicers, pulverizers, cutters, or slicers into pastes, suspensions, or cuts, into which cyclotetrasaccharide is incorporated by adding thereto and dissolving therein; or
(ii) extracted with solvents such as hot water and alcohol, and filtered, and if necessarily, further concentrated into liquids or pastes, followed by incorporating cyclotetrasaccharide into the resulting liquids or pastes by mixing to dissolve therein.

The compositions thus obtained are usually syrupy, pasty, or juicy disrupted products which have a relatively-high moisture content and require a relatively-low storage-temperature for a stable shelf life. The compositions can be directly dried and/or pulverized, or dried and/or pulverized after sterilization by heating to obtain powdery or small pieces of solid compositions. Conventional drying and pulverizing methods can be arbitrarily used in the present invention. The compositions can be prepared into powdery products by:
(i) successive drying *in vacuo,* air drying, drum drying, pulverizing, and sieving into powdery products;
(ii) successive spray drying, fluidized bed drying, and sieving into powdery products; or
(iii) successive mixing with an anhydrous saccharide such as maltose, trehalose, or cyclotetrasaccharide anhydride to effect dehydration, drying, and sieving into powdery products.

If necessarily, the powdery products can be arbitrarily granulated, tabletted, and encapsulated into appropriate forms for use. The solid compositions in a relatively-highly processed will acquire more improved activity for inhibiting the reduction of active oxygen eliminating activity, storage stability, and handelability. The solid compositions containing edible plant substances have features such that they effectively inhibit the deterioration of plant pigments contained in the edible plants as a material such as chlorophylls, carotenoids, anthocyanins, and flavonoids. Additionally, they satisfactorily retain the inherent color of pigments and mask undesirable tastes such as bitterness and astringency.

The compositions according to the present invention containing cyclotetrasaccharide and an edible plant substance or plant antioxidant, which inhibit the reduction of active oxygen eliminating activity, can be further mixed with one or more appropriate substances in a solid or liquid form such as essential minerals, edible fibers, saccharides for promoting the growth of bifid bacteria, vitamins, biologically active substances, and preservatives.

As the saccharides, trehalose, maltitol, and mannitol can be used. Essential minerals such as calcium, magnesium, phosphorus, iron, copper, zinc, and cobalt can be used in an appropriate amount.

As the edible fibers, pectin, alginic acid, carrageenan, gum Arabic, glucomannan, cyclodextrin, and pullulan can be used. Particularly, trehalose and/or pullulan and/or cyclodextrin are preferable and can be advantageously used with cyclotetrasaccharide to produce the compositions according to the present invention.

Examples of saccharides, which can be used to promote the growth of bifid bacteria, are lactosucrose, fructooligosaccharide, galactooligosaccharide, and isomaltooligosaccharide; and which can be arbitrarily used, if necessarily.

The vitamins advantageously used in the present invention include, for example, water soluble vitamins such as thiamin, riboflavin, L-ascorbic acid, rutin, hesperidin, naringin, niacin, pyridoxine, cyanocobalamin, and derivatives thereof; and lipid-soluble vitamins such as vitamin A, vitamin D, α-tocopherol, vitamin K, and derivatives thereof.

One or more biologically active substances such as hormones, antibiotics, cytokines, and propolis can be used, if necessarily. Adequate amounts of ethanol, acetic acid, lactic acid, and salt can be used as preservatives, if necessarily.

To improve the quality of the compositions of the present invention and products containing the compositions, adequate flavoring agents, coloring agents, taste-imparting agents, stabilizers, and excipients can be advantageously used.

The reduction inhibitory agent for active oxygen eliminating activity with cyclotetrasaccharide as an effective ingredient according to the present invention can be advantageously incorporated into plant substances with active oxygen eliminating activity to inhibit the reduction of active oxygen eliminating activity. The cyclotetrasaccharide content in the reduction inhibitory agent for active oxygen eliminating activity can be selected from those which exert a reduction inhibitory activity on the active oxygen eliminating activity of edible plant substances, usually, at least about 10 %, d.s.b., preferably, at least 20 %, d.s.b., and more preferably, at least 50 %, d.s.b. The present reduction inhibitory agent can be consisted of cyclotetrasaccharide as an effective ingredient and prepared with one or more other substances selected from the aforesaid essential minerals, fibers, saccharides for promoting the growth of bifid bacteria, vitamins, biological active substances, and preservatives, if necessarily.

The reduction inhibitory agent for active oxygen eliminating activity may be in any forms such as a syrup, powder, granule, or tablet. Any methods can be used in the present reduction inhibitory agent as long as the agent exerts a reduction inhibitory activity for plant active oxygen eliminating activity. For example, the reduction inhibitory agent for active oxygen eliminating activity can be used according to the aforesaid method, which is incorporated cyclotetrasaccharide into plant substances with active oxygen eliminating activity, to inhibit the reduction of the activity. Concretely, when the amount, method, and conditions using cyclotetrasaccharide as an effective ingredient to plant edible substances and/or plant antioxidants are similar to those used in the above method for inhibiting the reduction of plant active oxygen eliminating activity, the reduction inhibitory effect for plant active oxygen eliminating activity can be advantageously exerted to easily produce compositions which the reduction of the activity is inhibited.

The compositions thus obtained have at least five units/g, d.s.b., and preferably at least 10 units/g, d.s.b., of active oxygen eliminating activity as determined by the nitroblue tetrazolium (NBT) test in the later described Experiment 2. These compositions can be easily administered to subjects via appropriate routes, including orally, intubationally, percutaneously, and permucosally administrations to impart active oxygen eliminating activity without substantially deteriorating their quality. Intake of the compositions of the present invention containing plant edible substances enriches living bodies with plant functional ingredients such as vitamins, mineral, and edible fibers inherent to the plants, and enriches living bodies with active oxygen eliminating activity. Thus, the compositions of the present invention can be satisfactorily used to maintain and promote health, prevent aging and geriatric diseases, promote the treatment of incurable diseases, and inhibit carcinogenesis. The compositions can be used in food products, cosmetics, pharmaceuticals, and components and processing intermediates thereof.

To enrich nutritional value and improve quality and taste, the compositions of the present invention can be used for health food products and other food products in general. Examples of such are seasonings such as *"furikake"* (a seasoned fish meal), sauces, ketchup, *"yakiniku-no-tare*" (a sauce for Japanese grilled meat), curry roux, and instant soup mixes; Japanese and Western cakes such as *"mochi"* (a rice paste), *"dango"* (a ball of rice paste), candies, chewing gum, baked confections, snack confections, waffles, sponge cakes, buns, and breads; frozen desserts such as ice cream and ice candies; pastes such as fruit pastes, fruit sources, peanut pastes, and raw jams; pickles and pickled products; meat products such as hams and sausages; products of fish meats such as *"kamaboko"* (a steamed fish paste), *"chikuwa"* (a kind of fish paste), *"hanpen"* (a fish cake), and sausages; *"chinmi"* (a delicacy); *"tsukudani"* (a food boiled down in soy); dairy dishes; beverages such as vegetable juices, soymilk, fruit juices, and carbonated beverages; rice products such as rice gruel, porridge of rice and vegetables, and cooked rice with seasonings, meats, and vegetables; noodles such as frozen noodles, spaghetti, macaroni, and pastes; food products such as snacks, pudding and cake mixes, instant juices, instant soups, and frozen foods; and their components and processing intermediates.

The compositions according to the present invention can be used in cosmetics such as skin-beautifying agents, packs, creams, shampoos, hair rinses, hair tonics, bath salts, enzymic agents, and tooth pastes in the form of a liquid, paste, powder, or granule; and used as a component of a processing intermediate for these products.

The compositions according to the present invention can be used in pharmaceuticals such as an oral and/or intubation nutrient, curative, and interferon-inducing agent in the form of a liquid, paste, granule, tablet, or capsule. Additionally, they can be used to treat and prevent aging-related diseases in the form of an oral and/or intubation nutrient or therapeutic agent such as a coilunarium, nebula, digestive agent, stomachic, enzymic agent, ointment, cataplasm, and their components and processing intermediates.

The compositions of the present invention comprises food materials and can be used with less fear of causing toxicity. When administered orally, the compositions can be usually administered to subjects; on a dry solid basis at a dose of about 0.1 to 1,000 g/adult/day, preferably, about 0.2 to 500 g/adult/day, and more preferably, about 1 to 100 g/adult/day; and administered to subjects intubationally, percutaneously, and permucosally similarly as in the oral administration.

The following experiments explain the present invention in detail:

### Experiment 1

### Preparation of α-isomaltoglucosaccharide-forming enzyme and α-isomaltosyl-transferring enzyme

A liquid culture medium consisting of 4.0 % (w/v) of "PINE-DEX #4^{™}", a partial starch hydrolysate commercialized by Matsutani Chemical Ind., Tokyo, Japan, 1.8 % (w/v) of "ASAHIMEAST^{™}", a yeast extract commercialized by Asahi Breweries, Ltd., Tokyo, Japan, 0.1 % (w/v) of dipotassium phosphate, 0.06 % (w/v) of sodium phosphate dodecahydrate, 0.05 % (w/v) magnesium sulfate heptahydrate, and water was placed in 500-ml Erlenmeyer flasks in a respective amount of 100 ml, sterilized by autoclaving at 121 °C for 20 min, cooled, and then seeded with *Bacillus globisporus* C9 strain, FERM BP-7143, followed by culturing under rotary-shaking conditions at 27 °C and 230 rpm for 48 hours for a seed culture.

About 20 L of a fresh preparation of the same liquid culture medium as used in the above seed culture were placed in a 30-L fermentor, sterilized by heating, and then cooled to 27 °C and inoculated with one percent (v/v) of the seed culture, followed by culturing at 27 °C and pH 6.0-8.0 for 48 hours under aeration-agitation conditions. After completion of the culture, the resulting culture had about 0.45 unit/ml of the α-isomaltosylglucosaccharide-forming enzyme, about 1.5 units/ml of α -isomaltosyl-transferring enzyme, and about 0.95 unit/ml of cyclotetrasaccharide-forming activity. The supernatant was collected by centrifugation the culture at 10,000 rpm for 30 min. The resulting supernatant had about 0.45 unit/ml of the α-isomaltosylglucosaccharide-forming enzyme, i.e., a total enzymatic activity of about 8,110 units; and about 1.5 units/ml of α - isomaltosyl-transferring enzyme, i.e., a total enzymatic activity of about 26,900 units. This supernatant was salted out with 80 % saturated ammonium sulfate and allowed to stand at 4 °C for 24 hours, and the formed sediments were collected by centrifugation at 10,000 rpm for 30 min, dissolved in 10 mM phosphate buffer (pH 7.5), and dialyzed against a fresh preparation of the same buffer to obtain about 400 ml of a crude enzyme solution with 8,110 units of the α -isomaltosylglucosaccharide-forming enzyme, 24,700 units of α-isomaltosyl-transferring enzyme, and about 15,600 units of cyclotetrasaccharide-forming activity.

The activities of these enzymes were assayed as follows: The α-isomaltosylglucosaccharide-forming enzyme was assayed for enzymatic activity by dissolving maltotriose in 100 mM acetate buffer (pH 6.0) to give a concentration of two percents (w/v) for a substrate solution, adding a 0.5 ml of an enzyme solution to a 0.5 ml of the substrate solution, enzymatically reacting the mixture solution at 35 °C for 60 min, stopping the reaction by boiling for 10 min, and quantifying maltose among the isomaltosyl maltose and maltose formed in the reaction mixture on high-performance liquid chromatography (abbreviated as "HPLC" hereinafter). HPLC was carried out using "YMC Pack ODS-AQ303 column", a silica column commercialized by YMC Co., Ltd., Kyoto, Japan, at a column temperature of 40 °C and a flow rate of 0.5 ml/min of water, and using "RI-8012", a differential refractometer commercialized by Tosoh Corporation, Tokyo, Japan. One unit activity of the α-isomaltosylglucosaccharide-forming enzyme is defined as the enzyme amount that forms one micromole of maltose per minute under the above enzymatic reaction conditions.

The α-isomaltosyl-transferring enzyme was assayed for enzymatic activity by dissolving panose in 100 mM acetate buffer (pH 6.0) to give a concentration of two percents (w/v) for a substrate solution, adding a 0.5 ml of an enzyme solution to 0.5 ml of the substrate solution, enzymatically reacting the mixture solution at 35 °C for 30 min, stopping the reaction by boiling for 10 min, and quantifying glucose among the cyclotetrasaccharide and glucose formed in the reaction mixture by the glucose oxidase method. One unit activity of the α-isomaltosyl-transferring enzyme is defined as the enzyme amount that forms one micromole of glucose per minute under the above enzymatic reaction conditions.

The cyclotetrasaccharide-forming activity is assayed by dissolving "PINE-DEX #100^{™}", a partial starch hydrolysate commercialized by Matsutani Chemical Ind., Tokyo, Japan, in 50 mM acetate buffer (pH 6.0) to give a concentration of two percents (w/v) for a substrate solution, adding 0.5 ml of an enzyme solution to 0.5 ml of the substrate solution, enzymatically reacting the mixture solution at 35 °C for 60 min, stopping the reaction by boiling at 100 °C for 10 min, and then further adding to the resulting mixture one milliliter of 50 mM acetate buffer (pH 5.0) with 70 units/ml of "TRANSGLUCOSIDASE L AMANO^{™}", an α-glucosidase commercialized by Amano Pharmaceutical Co., Ltd., Aichi, Japan, and 27 units/ml of a glucoamylase commercialized by Nagase Biochemicals , Ltd., Kyoto, Japan, and incubated at 50 °C for 60 min, inactivating the remaining enzymes by heating at 100 °C for 10 min, and quantifying cyclotetrasaccharide on the above HPLC. One unit of cyclotetrasaccharide-forming activity is defined as the enzyme amount that forms one micromole of cyclotetrasaccharide per minute under the above enzymatic reaction conditions.

### Experiment 2

### Preparation of cyclotetrasaccharide

About 100 L of a four percents (w/v) aqueous solution of corn phytoglycogen, commercialized by Q. P. Corporation, Tokyo, Japan, was prepared, adjusted to pH 6.0 and 30 °C, and then admixed with 0.15 ml/g starch of α-isomaltosylglucosaccharide-forming enzyme and α-isomaltosyl-transferring enzyme from *Bacillus globisporus* C9 strain obtained by the method in Experiment 1, followed by the incubation for 48 hours. The reaction mixture was heated at 100 °C for 10 min to inactivate the remaining enzymes and a portion of the reaction mixture was sampled and then quantified on HPLC for the formation yield of cyclotetrasaccharide, revealing that it contained about 84 % cyclotetrasaccharide on a saccharide composition basis. HPLC was carried out by using "SHODEX KS-801 column", commercialized by Showa Denko K. K., Tokyo, Japan, at a column temperature of 60 °C and a flow rate of 0.5 ml/min of water, and using "RI-8012", a differential refractometer commercialized by Tosoh Corporation, Tokyo, Japan. The reaction mixture was adjusted to pH 5.0 and 45 °C , and then incubated for 24 hours after admixed with 1,500 units/ g starch of "TRANSGLUCOSIDASE AMANO^{™}", an α-glucosidase commercialized by Amano Pharmaceutical Co., Ltd., Aichi, Japan, and 75 units/g starch of "XL-4^{™}", a glucoamylase commercialized by Nagase Biochemicals, Ltd., Kyoto, Japan, to decompose the remaining reducing oligosaccharides. The resulting culture was adjusted to pH 5.8 by the addition of sodium hydroxide and incubated at 90 °C for one hour to inactivate the remaining enzymes and filtered to remove insoluble substances. The filtrate was concentrated using "HOLLOSEP HR5155PI^{™}", a reverse osmosis membrane by Toyobo Co., Ltd., Osaka, Japan, to give a concentration of about 16 %, d.s.b., and the concentrate was in a conventional manner decolored, desalted, filtered, and concentrated to obtain about 6.2 kg of an aqueous saccharide solution with a solid content of about 3,700 g. The aqueous saccharide solution was fed to a column packed with about 225 L of "AMBERLITE CR-1310 (Na⁺-form)^{™}", an ion-exchange resin commercialized by Japan Organo Co., Ltd., Tokyo, Japan, and chromatographed at a column temperature of 60 °C and a flow rate about 45 L/h. While the saccharide composition of elute from the column was monitored by the aforesaid HPLC, fractions with a purity of at least 98 % of cyclotetrasaccharide were collected, and in a conventional manner desalted, decolored, filtered, and concentrated to obtain about 7.5 kg of an aqueous saccharide solution with a solid content of about 2,500 g. HPLC measurement for saccharide composition of the aqueous saccharide solution revealed that it contained cyclotetrasaccharide with a purity of about 99.5%. The obtained aqueous saccharide solution containing cyclotetrasaccharide was concentrated by evaporation to give a concentration of about 50 %, d.s.b. About five kilograms of the concentrate was placed in a cylindrical plastic vessel and then crystallized by lowering the temperature of the concentrate from 65 °C to 20 °C for about 20 hours under gentle rotatory conditions. The crystallized concentrate was separated by a centrifugal filter to obtain 1,360 g by wet weight of a crystal of cyclotetrasaccharide. Additionally, the crystal was dried at 60 °C for three hours to obtain 1,170 g of a crystalline powder of cyclotetrasaccharide. HPLC measurement of the crystalline powder revealed that it contained cyclotetrasaccharide with a remarkably-high purity of at least 99.9 %.

### Experiment 3

### Influence of saccharides on reduction inhibitory activity for active oxygen eliminating activity

Fresh raw carrots were disrupted by a mixer, and saccharides were respectively added to the mixture and dissolved therein to give a concentration of 10 %, d.s.b. The solutions were dried under a reduced pressure at 40 °C for two days, dried *in vacuo* at the same temperature for 24 hours, and pulverized by a mixer into powdery carrot compositions. The saccharides used were the cyclotetrasaccharide obtained in Experiment 2 and commercialized reagent-grade glucose, mannitol, sorbitol, maltose, sucrose, trehalose, and pullulan. An about 100 g each of the compositions was placed and sealed in a 500-ml polystyrene container, and stored at 40 °C for seven days. The active oxygen eliminating activities of the compositions before and after storage were compared with each other. The active oxygen eliminating activity was assayed by the nitroblue tetrazolium (NBT) test by Toshio IMANARI et al. in *Igakuno Ayumi,* Vol. 101, pp. 496-497 (1977), and the level of superoxide, formed in a xanthine-xanthine oxidase system, was assayed by quantifying calorimetrically the content of formazan formed by reducing NBT. As a control, refined water was used as a test solution. One unit activity of active oxygen eliminating activity was defined as that inhibited 50 % formazan formation as compared with the control. Table 1 shows the composition of the powdery compositions and the active oxygen eliminating activity per gram of the compositions before and after storage. As evident from Table 1, among the saccharides tested the composition with cyclotetrasaccharide or trehalose showed the highest residual percentage for active oxygen eliminating activity, revealing that cyclotetrasaccharide exerts a remarkable effect on the reduction inhibitory activity for active oxygen eliminating activity similar to trehalose. Thus cyclotetrasaccharide is preferable as an effective ingredient for a reduction inhibitory agent for active oxygen eliminating activity. Next to cyclotetrasaccharide and trehalose, pullulan showed a relatively-high reduction inhibitory activity for active oxygen eliminating activity.

**Table 1**

| Saccharide | Composition of powdery composition(%) | | | Saccharide content(d.s.b.) (%) | Activity (unit/g) | | Percentage of residual activity (%) |
|---|---|---|---|---|---|---|---|
| | Carrot | Saccharide | Moisture | | Before storage | After storage | |
| None | 93.4 | 0.0 | 6.6 | 0.0 | 870 | 100 | . 11 |
| Cyclotetrasaccharide | 47.9 | 45.7 | 6.4 | 48.8 | 590 | 390 | 66 |
| Glucose | 48.8 | 46.4 | 4.8 | 48.7 | 580 | 170 | 29 |
| Mannitol | 49.8 | 47.5 | 2.7 | 48.8 | 520 | 59 | 11 |
| Sorbitol | 48.2 | 46.0 | 5.8 | 48.8 | 560 | 26 | 5 |
| Maltose | 48.1 | 45.8 | 6.1 | 48.8 | 550 | 180 | 33 |
| Sucrose | 48.2 | 46.0 | 5.8 | 48.8 | 660 | 180 | 27 |
| Trehalose | 47.9 | 45.7 | 6.4 | 48.8 | 580 | 380 | 66 |
| Pullulan | 47.7 | 45.5 | 6.8 | 48.8 | 710 | 360 | 51 |

### Experiment 4

### influence of saccharides on reduction inhibitory activity for active oxygen eliminating activity

Fresh raw carrots were disrupted as Experiment 3, and saccharides were respectively added to the mixture and dissolved therein to give a concentration of 10 %, d.s.b. The solutions were dried under a reduced pressure at 40 °C for one day, dried *in vacuo* at the same temperature for 17 hours, and pulverized by a mixer into powdery carrot compositions. The saccharides used were the cyclotetrasaccharide obtained in Experiment 2 and commercialized reagent-grade glucose, maltose, and trehalose. About 100 g each of the compositions was placed and sealed in a 500-ml polystyrene container, and stored at 40 °C for seven or fourteen days. The active oxygen eliminating activities of the compositions before and after storage were compared with each other. The active oxygen eliminating activity was assayed by the electron spin resonance (ESR) test by Toshihiko OZAWA in *BUNSEKI*, Vol. 2, pp. 68-74 (2000), and the level of superoxide, formed in a hypoxanthine-xanthine oxidase system, was assayed by the spin-trapping with 5,5-dimethyl-1-pyrroline-N-oxide in ESR. A standard was prepared by diluting a commercialized superoxide dismutase, Sigma Chemical Co., Tokyo, Japan, in 0.1 M phosphate buffer (pH 7.4) to give either concentrations of 0.5-13.5 units/ml. The activity of active oxygen eliminating activity was defined with reference to the standard. Table 2 shows the compositions of the powdery compositions and the active oxygen eliminating activity per gram each of the compositions before and after storage. As evident from Table 2, among the saccharides tested the composition with cyclotetrasaccharide or trehalose showed the highest residual percentage for active oxygen eliminating activity, revealing that cyclotetrasaccharide exerted a remarkable effect on the reduction inhibitory activity for active oxygen eliminating activity similar to trehalose. Thus cyclotetrasaccharide is preferable as an effective ingredient for a reduction inhibitory agent for active oxygen eliminating activity.

**Table 2**

| Saccharide | Composition of powdery composition(%) | | | Saccharide content(d.s.b.) (%) | Activity (unit/g) | | |
|---|---|---|---|---|---|---|---|
| | Carrot | Saccharide | Moisture | | Before storage | After one week storage | After two weeks storage |
| None | 96.0 | 0.0 | 4.0 | 0.0 | 375 | 113(30%) | 87(23%) |
| Cyclotetrasaccharide | 46.1 | 47.7 | 6.3 | 50.9 | 186 | 88(47%) | 66(36%) |
| Glucose | 46.3 | 48.0 | 5.7 | 50.9 | 172 | 48(28%) | 36(21%) |
| Maltose | 46.5 | 48.5 | 5.0 | 51.1 | 236 | 56(24%) | 53(22%) |
| Trehalose | 46.2 | 47.7 | 6.0 | 50.8 | 198 | 91(46%) | 73(37%) |

| | | | | | | | |
|---|---|---|---|---|---|---|---|
| Note: Numerals in parentheses mean percentages of residual activities. | | | | | | | |

### Experiment 5

### Influence of cyclotetrasaccharide concentration of reduction inhibitory activity for active oxygen eliminating activity

Similarly as in Experiment 3, cyclotetrasaccharide was respectively mixed with and dissolved in disrupted carrots to give concentrations of 0-20 %, d.s.b. The resulting mixtures were dried *in vacuo* at 40°C for 24 hours, and pulverized into powdery carrot compositions. Similarly as in Experiment 3, each powdery composition was placed and sealed in a polystyrene container, and stored at 40°C for seven days for evaluating the reduction inhibitory activity for active oxygen activity. The active oxygen eliminating activity of one gram each of powdery compositions before and after storage was measured by the method in Experiment 3. Table 3 shows the results. Table 3 shows that at least about one percent, preferably, at least about five percents, and more preferably, at least about 20 % of cyclotetrasaccharide, d.s.b., exerted a desired effective reducing inhibitory activity for active oxygen eliminating activity.

**Table 3**

| Percentage of cyclotetrasaccharide added to carrot | Composition of powdery composition(%) | | | Cyclotetrasaccharide content (d.s.b.) (%) | Activity (unit/g) | | Percentage of residual activity (%) |
|---|---|---|---|---|---|---|---|
| | Carrot | Saccharide | Moisture | | Before storage | After storage | |
| 0.0 | 95.3 | 0.0 | 4.7 | 0.0 | 940 | 330 | 35 |
| 0.2 | 94.3 | 1.0 | 4.7 | 1.0 | 930 | 390 | 42 |
| 0.5 | 90.4 | 4.7 | 4.9 | 4.9 | 820 | 470 | 57 |
| 1.0 | 85.9 | 8.9 | 5.2 | 9.4 | 740 | 450 | 61 |
| 2.5 | 75.3 | 19.6 | 5.1 | 20.7 | 700 | 460 | 66 |
| 5.0 | 62.3 | 32.4 | 5.3 | 34.2 | 550 | 340 | 62 |
| 10.0 | 46.1 | 48.1 | 5.8 | 51.1 | 420 | 290 | 69 |
| 20.0 | 30.3 | 63.1 | 6.6 | 67.6 | 390 | 280 | 64 |

### Experiment 6

### Influence of cyclotetrasaccharide on reducing inhibitory activity for active oxygen eliminating activity of vegetables

Vegetables were treated similarly as in Experiment 3, and cyclotetrasaccharide was respectively added to the disrupted vegetables to give a concentration of 10 %, d.s.b. The resulting mixtures were dried *in vacuo* at 45 °C for 20 hours, and pulverized into powdery vegetable compositions. Similarly as in Experiment 3, each composition was respectively placed in a container and stored at 40 °C for six days. The active oxygen eliminating activity of one gram each of the compositions before and after storage was compared with that of the composition prepared without cyclotetrasaccharide. Table 4 shows the compositions of the powdery compositions and their active oxygen eliminating activities. A reduction inhibitory activity for active oxygen eliminating activity by incorporating cyclotetrasaccharide was observed in many vegetables such as carrot, onion, Japanese radish, cabbage, spinach, cucumber, and pumpkin.

**Table 4**

| Vegetable (moisture(%)) | Composition of powdery composition(%) | | | Cyclotetrasaccharide content(d.s.b.) (%) | Activity (unit/g) | | Percentage of residual activity (%) |
|---|---|---|---|---|---|---|---|
| | Carrot | Saccharide | Moisture | | Before storage | After storage | |
| Carrot | 94.5 | 0.0 | 5.5 | 0.0 | 1,200 | 370 | 31 |
| (90.1) | 46.0 | 46.8 | 7.2 | 50.4 | 560 | 460 | 75 |
| Onion | 93.2 | 0.0 | 6.8 | 0.0 | 440 | 210 | 48 |
| (89.3) | 47.7 | 44.1 | 8.2 | 48.0 | 220 | 160 | 73 |
| Japanese radish | 92.7 | 0.0 | 7.3 | 0.0 | 1,100 | 630 | 57 |
| (94.0) | 34.7 | 58.6 | 6.7 | 62.8 | 400 | 320 | 80 |
| Cabbage | 94.8 | 0.0 | 5.2 | 0.0 | 4,100 | 2,500 | 63 |
| (93.8) | 36.0 | 57.3 | 6.7 | 61.4 | 1,500 | 1,300 | 87 |
| Spinach | 93.2 | 0.0 | 6.8 | 0.0 | 3,500 | 1,300 | 36 |
| (94.1) | 34.1 | 58.6 | 7.3 | 63.2 | 1,300 | 900 | 69 |
| Eggplant | 91.5 | 0.0 | 8.5 | 0.0 | 38,000 | 31,000 | 82 |
| (93.7) | 35.0 | 55.3 | 9.7 | 61.2 | 13,000 | 11,000 | 85 |
| Cucumber | 92.7 | 0.0 | 7.3 | 0.0 | 4,600 | 1,300 | 30 |
| (95.0) | 30.1 | 60.6 | 9.3 | 66.8 | 1,500 | 890 | 59 |
| Pumpkin | 93.2 | 0.0 | 6.8 | 0.0 | 1,600 | 610 | 38 |
| (84.0) | 56.3 | 35.1 | 8.6 | 38.4 | 900 | 450 | 50 |

### Experiment 7

### Reduction inhibitory activity for active oxygen eliminating activity by cyclotetrasaccharide to superoxide dismutase and antioxidants

In one milliliter of 30 % (w/v) cyclotetrasaccharide solution, which is prepared by using cyclotetrasaccharide obtained in Experiment 2, was dissolved five micrograms (µg) of superoxide dismutase from a horseradish commercialized by Sigma Chemical Co., St. Louis, USA, 10 mg of α-glucosyl hesperidin commercialized by Hayashibara Biochemical Laboratories, Inc., Okayama Japan, 10 mg of α-glucosyl rutin commercialized by Hayashibara Biochemical Laboratories, Inc., Okayama, Japan, 250 µg of gallic acid commercialized by Wako Pure Chemical Industries, Ltd., Tokyo, Japan, or 50 µg of catechin to obtain a liquid composition. Each of the compositions was placed in a glass test tube, sealed with a rubber cap, and stored under a dark condition. As a control, water was used in place of the cyclotetrasaccharide solution. Table 5 shows the active oxygen eliminating activity before and after storage. As shown in Table 5, it was confirmed that cyclotetrasaccharide exerts an effective reducing inhibitory activity for active oxygen eliminating activity of plant antioxidants such as superoxide dismutase, α-glucosyl hesperidin, α-glucosyl rutin, gallic acid, and catechin. When a powdery crystalline cyclotetrasaccharide hydrate and either of the powdery antioxidants into a powders mixture were mixed and subjected to storage test similarly as above, almost no change of the active oxygen eliminating activity was observed. Based on the results, it was revealed that a reducing inhibitory agent for active oxygen eliminating activity, which comprises cyclotetrasaccharide as an effective ingredient, could scarcely exert the desired activity only when mixed in a powdery form with powdery substances having active oxygen eliminating activity, but exert the desired activity when cyclotetrasaccharide in a melted state is allowed to contact with and incorporate into the substances, or when cyclotetrasaccharide is incorporated into the substances in an aqueous system.

The following Examples explain the present invention; Examples A explains the reducing inhibitory agent for active oxygen eliminating activity of the present invention, and Examples B explains the composition of the present invention where the reduction of active oxygen eliminating activity is inhibited:

**Table 5**

| Content of antioxidant | Storage Condition | Cyclotetrasaccharide concentration (%) | Activity (unit/mg antioxidant) | | Residual activity (%) |
|---|---|---|---|---|---|
| | | | Before storage | After one storage | |
| Superoxide dismutase | 60 °C | 0 | 10,000 | 4,600 | 46 |
| (5 µg/ml) | 17 hours | 30 | 12,000 | 7,300 | 61 |
| α-Glucosyl hesperidin | 80 °C | 0 | 3.8 | 1.0 | 26 |
| (10 mg/ml) | 5 days | 30 | 4.0 | 2.1 | 53 |
| α-Glucosyl rutin | 80 °C | 0 | 25 | 18 | 72 |
| (10 mg/ml) | 5 days | 30 | 26 | 22 | 85 |
| Gallic acid | 80 °C | 0 | 590 | 390 | 66 |
| (250 µg/ml) | 5 days | 30 | 600 | 450 | 75 |
| Catechin | 25 °C | 0 | 470 | 2.2 | 0 |
| (50 µg/ml) | 20 hours | 30 | 450 | 240 | 53 |

### Example A-1

### Reduction Inhibitory Agent for Active oxygen Eliminating Activity

Corn starch was prepared into an about 20 % starch suspension which was mixed with 0.1 % calcium carbonate, adjusted to pH 6.5, mixed with 0.3 % per gram starch of "TERMAMYL 60L^{™}", an α-amylase commercialized by Novo Industri A/S, Copenhagen, Denmark, reacted at 95 °C for 15 min, autoclaved at 120 °C for 20 min, and promptly cooled to about 35 °C to obtain a liquefied solution with DE of about four. Thereafter, the liquefied solution was admixed with 0.2 ml per gram starch of an enzyme solution containing α-isomaltosylglucosaccharide-forming enzyme and α -isomaltosyl-transferring enzyme from *Bacillus globisporus* C9 strain obtained by the method in Experiment 1, and 10 units per gram solid starch of cyclomaltodextrin glucanotransferase commercialized by Hayashibara Biochemical Laboratories, Inc., Okayama Japan, adjusted to pH 6.0, and reacted at 35 °C for 48 hours. After the reaction mixture was incubated at 95 °C for 30 min, it was adjusted to pH 5.0 and 50 °C , and then admixed with 300 unites per gram solid substance of "TRANSGLUCOSIDASE L AMANO^{™}", an α-glucosidase commercialized by Amano Pharmaceutical Co., Ltd., Aichi, Japan, and reacted for 24 hours. The reaction mixture was admixed with 30 units per gram solid substance of "GLUCOZYME^{™}", a glucoamylase, commercialized by Nagase Biochemicals, Ltd., Kyoto, Japan, reacted for 17 hours, incubated at 95 °C for 30 min, cooled down, and filtrated. The obtained filtrate was in a conventional manner decolored by using an activated carbon, desalted by using H- and OH-types ion-exchanging resins, purified, and concentrated to obtain a cyclotetrasaccharide content syrup with the concentration of 60 % which contains 34.2 % of glucose, 62.7 % of cyclotetrasaccharide, and 3.1 % of other saccharides, d.s.b.

The cyclotetrasaccharide content syrup was fractionated by using "AMBERLITE CR-1310 (Na⁺-form)^{™}", a strong acidic cation-exchange resin commercialized by Japan Organo Co., Ltd., Tokyo, Japan. The resin was packed in four stainless steal jacketed-columns, 5.4 cm in diameter and five meters in length. The columns were connected in series to a total resin length of 20 m. While the column temperature was kept at 60 °C, the cyclotetrasaccharide content syrup was fed to the columns at a ratio of five percents (v/v) resin and fractionated by feeding a warm water of 60 °C at SV (space velocity) 0.13. While the saccharide composition of elute from the columns was monitoring by HPLC, fractions with cyclotetrasaccharide were collected and purified to obtain a cyclotetrasaccharide high content solution with the purity of about 98 % on d.s.b.

The cyclotetrasaccharide high content solution was concentrated to the concentration of about 70 %, and then placed in a crystallizer, admixed with about two percents of a seed crystal of crystalline cyclotetrasaccharide hydrate, and cooled to obtain a massecuite with a crystallization degree of about 45 %. The massecuite was sprayed from a nozzle on a dried tower at relatively-high pressure of 150 kg/cm². At the same time, a hot wind with a temperature of 85 °C was blown from the upper of the dried tower, and a crystalline powder was collected on a gauze conveyer for a transfer, and taken out by gradually transferring to outside of the dried tower while blowing a warm wind with a temperature of 45 °C from under the conveyer. The powder was packed to an aged tower, and crystallized and dried by aging for 10 hours while blowing a warm wind to obtain a powdery crystalline saccharide in a yield of about 20 % to the material starch, d.s.b. The powdery saccharide is a high-purity crystalline cyclotetrasaccharide hydrate, and it is preferable as a reduction inhibitory agent for active oxygen eliminating activity and can be arbitrarily used to inhibit the reduction of active oxygen eliminating activity of plant edible substances.

### Example A-2

### Reduction Inhibitory Agent for Active oxygen Eliminating Activity

One hundred parts by weight of a high-purity crystalline cyclotetrasaccharide hydrate, obtained by the method in Example A-1, was homogeneously mixed with one part by weight of pullulan to produce a solid reduction inhibitory agent for active oxygen eliminating activity. The product can be arbitrarily used in a reduction inhibitory agent for active oxygen eliminating activity of plant edible substances.

### Example A-3

### Reduction Inhibitory Agent for Active oxygen Eliminating Activity

In one hundred parts by weight of a cyclotetrasaccharide content syrup with the concentration of 60 %, obtained by the method in Example A-1, was dissolved by homogeneously mixing to 0.5 parts by weight of pullulan, and 0.5 parts by weight of a readily-water-soluble cyclodextrin into a syrupy reduction inhibitory agent for active oxygen eliminating activity. The product can be arbitrarily used in a reduction inhibitory agent for active oxygen eliminating activity of plant edible substances.

### Example A-4

### Reduction Inhibitory Agent for Active oxygen Eliminating Activity

In one hundred parts by weight of a high-purity crystalline cyclotetrasaccharide hydrate, obtained by the method in Example A-1, was dissolved by homogeneously mixing 100 parts by weight of crystalline trehalose hydrate, one part by weight of pullulan, and one part by weight of a readily-water-soluble cyclodextrin into a powdery reduction inhibitory agent for active oxygen eliminating activity. The product can be arbitrarily used in a reduction inhibitory agent for active oxygen eliminating activity of plant edible substances.

### Example B-1

### Composition Containing Spinach

Fresh spinach was disrupted by a mixer, and in 10 parts by weight of the resulting disruptant were mixed by dissolving one part by weight of a reduction inhibitory agent for active oxygen eliminating activity obtained by the method in Example A-4 and 0.1 part by weight of α-glucosyl rutin in an appropriate volume of water. The resulting mixture was heated at 100°C for 10 min, dried with air heated to 40°C for two hours, dried *in vacuo* at 40°C for 16 hours, and powdered by a pulverizer to obtain a composition containing spinach having an active oxygen eliminating activity of about 1,200 units per gram composition. The composition satisfactorily retained the inherent color of spinach and stimulated the appetite. Intake of the composition enriches living bodies with functional ingredients such as vitamins, minerals, and edible fibers inherent to the spinach, and imparts the active oxygen eliminating activity to the living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-2

### Composition Containing Cabbage

Cabbage was cut, branched, and sliced by a cutter into fine strips with about five millimeters in width. Ten parts by weight of the fine strips was mixed with one part by weight of a reduction inhibitory agent for active oxygen eliminating activity obtained by the method in Example A-3, and 0.05 part by weight of α-glucosyl rutin, and the mixture was allowed to stand at ambient temperature for two hours, then dried by air heated to 40 °C for two hours, dried *in vacuo* at 40 °C for 16 hours to obtain a composition containing fine strips of cabbage having an active oxygen eliminating activity of about 1,300 units per gram composition. The composition retained satisfactorily the inherent color of cabbage and stimulated your appetite. Intake of the composition enriches living bodies with functional ingredients such as vitamins, and edible fibers inherent to the cabbage, and imparts the active oxygen eliminating activity to the living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-3

### Composition Containing Eggplant

A fresh eggplant was sliced, and the sliced eggplant was washed with water. One part by weight of a reduction inhibitory agent for active oxygen eliminating activity, obtained by the method in Example A-1, was dissolved in an appropriate volume of water and mixed with 10 parts by weight of the sliced eggplant, and the resulting mixture was dried by air heated to 70 °C for two days to obtain a composition containing sliced eggplant having an active oxygen eliminating activity of about 11,000 units per gram composition. The composition retained the inherent color of eggplant's skin and stimulated the appetite. Intake of the composition enriches living bodies with functional ingredients such as vitamins, minerals, and edible fibers inherent to the eggplant, and imparts the active oxygen eliminating activity to the living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-4

### Composition Containing Carrot

A fresh carrot was branched and sliced by a slicer. One part by weight of a reduction inhibitory agent for active oxygen eliminating activity, obtained by the method in Example A-4, was dissolved in an appropriate volume of water and mixed with 10 parts by weight of the sliced carrot. The mixture was dried with air heated to 50 °C for 16 hours to obtain a composition containing sliced carrots having an active oxygen eliminating activity of about 450 units per gram composition. The composition satisfactorily retained the color inherent to carrot, stimulated the appetite, and had a relatively-low hygroscopicity and a satisfactory shelf-life. Intake of the composition enriches living bodies with functional ingredients such as vitamins, minerals, and edible fibers inherent to carrot, and imparts the active oxygen eliminating activity to the living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-5

### Composition Containing Hizikia fusiforme

Dried *Hizikia fusiforme* was rehydrated in water and disrupted with a cutter. In 10 parts by weight of the disrupted mixture was dissolved by mixing five parts by weight of a reduction inhibitory agent for active oxygen eliminating activity obtained by the method in Example A-2. The solution was dried by air heated to 50 °C for eight hours, dried *in vacuo* at 40 °C, and pulverized into a composition containing *Hizikia fusiforme* having an active oxygen eliminating activity of about 24 units per gram composition. Intake of the composition enriches living bodies with functional ingredients such as vitamins, minerals, and edible fibers inherent to *Hizikia fusiforme,* and imparts the active oxygen eliminating activity to the living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-6

### Composition Containing Chinese Mushroom

A dried Chinese mushroom was rehydrated in water, heated at 100 °C for 15 min, and disrupted by a cutter. In 100 parts by weight of the disrupted mixture was dissolved by mixing 0.1 part by weight of a tea extract and 10 parts by weight of a reduction inhibitory agent for active oxygen eliminating activity obtained by the method in Example A-3, and the resulting mixture was dried at 50 °C for eight hours, dries *in vacuo* at 40 °C, and pulverized to obtain a composition containing a powdery Chinese mushroom having an active oxygen eliminating activity of about 560 units per gram composition. Intake of the composition enriches living bodies with functional ingredients such as vitamins, minerals, and edible fibers inherent to Chinese mushroom, and imparts the active oxygen eliminating activity to the living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-7

### Composition Containing Citron

Citron peel was disrupted by a cutter, and five parts by weight of the disrupted peel was dissolved in an appropriate volume of water and mixed with one part by weight of a reduction inhibitory agent for active oxygen eliminating activity, obtained by the method in Example A-1. After preliminary freezing, the resulting mixture was freeze-dried for three days, and disrupted to obtain a composition containing a powdery citron having an active oxygen eliminating activity of about 12 units per gram composition. The composition retains satisfactorily the color and flavor inherent to citron. Intake of the composition enriches living bodies with functional ingredients such as vitamins, minerals, and edible fibers inherent to the citron, and imparts the active oxygen eliminating activity to living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-8

### Composition Containing Ginger

A fresh ginger was disrupted by a mixer. One part by weight of a reduction inhibitory agent for active oxygen eliminating activity obtained by the method in Example A-2 was dissolved in an appropriate volume of water and mixed with five parts by weight of the disrupted mixture. After preliminary freezing, the resulting mixture was frozen and dried for three days, and disrupted by a crusher to obtain a composition containing a powdery ginger having an active oxygen eliminating activity of about 120 units per gram composition. Intake of the composition enriches living bodies with functional ingredients such as vitamins, minerals, and edible fibers inherent to ginger, and imparts the active oxygen eliminating activity to the living bodies. The composition can be satisfactorily used to maintain and promote your health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-9

### Composition Containing Aojiso (a Beefsteak Plant)

*Aojiso* leaves were branched and disrupted by a mixer. One part by weight of a reduction inhibitory agent for active oxygen eliminating activity obtained by the method in Example A-1 and 0.5 part by weight of α-glucosyl rutin were dissolved in an appropriate volume of water and mixed with five parts by weight of the disrupted mixture was dissolved by mixing, then mixed with 35 parts by weight of crystalline cyclotetrasaccharide anhydride as a desiccant. The resulting mixture was allowed to stand at ambient temperature for one day and disrupted by a crusher to obtain a composition containing a powdery *aojiso* having an oxygen eliminating activity of about 230 units per gram composition. The composition satisfactorily retained the color, flavor and taste inherent to *aojiso,* and stimulated the appetite. Intake of the composition enriches living bodies with functional ingredients such as vitamins, minerals, and edible fibers inherent to the *aojiso,* and imparts the active oxygen eliminating activity to living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example 10

### Composition Containing Mugwort

According to a conventional manner, mugwort was placed into boiling water with an adequate amount of salt to remove harshness, dehydrated softly and disrupted by a crusher. One point three (1.3) parts by weight of a reduction inhibitory agent for active oxygen eliminating activity obtained by the method in Example A-5 and 0.05 part by weight of α-glucosyl rutin were dissolved in an appropriate volume of water and mixed with five parts by weight of the disrupted mixture. The resulting mixture was dried by air heated to 50 °C for four hours, dried *in vacuo* at 40 °C for 16 hours, and disrupted to obtain a composition containing a powdery mugwort having an active oxygen eliminating activity of about 780 units per gram composition. The composition satisfactorily retained the color, flavor and taste inherent to mugwort, and stimulated the appetite. Intake of the composition enriches living bodies with functional ingredients such as vitamins, minerals, and edible fibers inherent to mugwort, and imparts the active oxygen eliminating activity to the living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-11

### Composition Containing Dokudami

A 0.5 part by weight of a dried *dokudami* was mixed with 10 parts by weight of water, and heated to boiling for 90 min. A supernatant of the infused solution was collected by a basket-type centrifuge and boiled down to obtain four parts by weight of about five percents solution. Two parts by weight of he concentrate was mixed with 0.5 part by weight of a reduction inhibitory agent for active oxygen eliminating activity obtained by the method in Example A-3, and the resulting solution was spray-dried in a conventional manner to obtain a composition containing a powdery *dokudami* extract having an active oxygen eliminating activity of about 1,400 units per gram composition. The composition had a lesser unsatisfactory smell and taste inherent to *dokudami* and could be more easily swallowable than conventional ones. Intake of the composition enriches living bodies with functional ingredients such as vitamins, minerals, an edible fibers inherent to the *dokudami,* and imparts the active oxygen eliminating activity to living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-12

### Composition Containing Aloe

A fresh aloe was disrupted by a mixer. One part by weight of a reduction inhibitory agent for active oxygen eliminating activity obtained by the method in Example A-1 was dissolved in an appropriate volume of water and mixed with 10 parts by weight of the disrupted aloe and 0.1 part by weight of a tea extract. The resulting mixture was dried by air, heated to 70 °C for two days, and disrupted by a crusher to obtain a composition containing a powdery aloe having an active oxygen eliminating activity of 5,800 units per gram composition. The composition had a lesser astringency of the aloe and was more easily swallowable than conventional ones. Intake of the composition enriches living bodies with functional ingredients such as vitamins, minerals, and edible fibers inherent to aloe, and imparts the active oxygen eliminating activity to living bodies. The composition can be satisfactorily used to maintain and promote health, prevent aging and geriatric diseases, promote the treatment of incurable diseases, and prevent carcinogenesis. Thus the composition can be arbitrarily used as a food product, cosmetic, pharmaceutical, or their material or processing intermediates.

### Example B-13

### Composition Containing Chinese Cabbage Preserved with Seasonings

A Chinese cabbage preserved with seasonings was cut by a chopping machine. One part by weight of a reduction inhibitory agent for active oxygen eliminating activity obtained by the method in Example A-1 was dissolved in an appropriate volume of water and mixed with 10 parts by weight of the cut product, and the mixture was dried with air heated to 55 °C for 16 hours to obtain a composition containing small pieces of Chinese cabbage, preserved with seasonings, having an active oxygen eliminating activity of about 5,400 units per gram composition. The product retained the color tint inherent to Chinese cabbage. When tasted, the satisfactory taste and flavor spread throughout the mouth and stimulated the appetite. Intake of this composition enriches living bodies with functional ingredients such as vitamins, minerals, and edible fibers inherent to Chinese cabbage, and augments the active oxygen eliminating activity to the living bodies. The compositions can be satisfactorily used to maintain and promote health, prevent aging and geriatric disease, promote the treatment of incurable diseases, and prevent carcinogenesis. Thus, the composition can be used as a food product, cosmetic, pharmaceutical, or a component of processing intermediate thereof. The compositions can also be used as food products such as *furikake,* premix for *onigiri* (a rice ball), and soup for Chinese noodles, and as a health food, cosmetic, pharmaceutical, or components and intermediates thereof.

### Example B-14

### Composition of Brassica rapa

A pickled *Brassica rapa* was cut by a chopping machine. One part by weight of a reduction inhibitory agent for active oxygen eliminating activity obtained by the method in Example A-1 was dissolved in an appropriate volume of water and mixed with 10 parts by weight of the cut product. The mixture was dried in air, heated to 55 °C for 16 hours to obtain a composition containing small pieces of pickled *Brassica rapa* having an active oxygen eliminating activity of about 2600 units per gram composition. The product retained the color tint inherent to picked *Brassica rapa.* When tasted, the satisfactory taste and flavor spread throughout the mouth and stimulated the appetite. Intake of this composition enriches living bodies with functional ingredients such as vitamins, minerals and edible fibers inherent to pickled *Brassica rapa,* and augments the active oxygen eliminating activity to the living bodies. The composition can be used to maintain and promote health, prevent aging and geriatric diseases, promote treatment of incurable diseases, and prevent carcinogenesis. Thus, the composition can be used as a food product, cosmetic, pharmaceutical, or components or processing intermediates thereof.

### Example B-15

### Composition of Korean Pickled Chinese Cabbage

Korean pickled Chinese cabbage was cut by a chopping machine. One part by weight of a reduction inhibitory agent for active oxygen eliminating activity obtained by the method of Example A-4 was dissolved in an appropriate volume of water and mixed with 10 parts by weight of the cut product. The mixture was air dried and heated to 55 °C for 16 hours to produce a composition containing small pieces of pickled Chinese cabbage having an active oxygen eliminating activity of about 1600 units per gram composition. The product retained the color tine of Korean pickled Chinese cabbage. When tasted, the satisfactory taste and flavor spread throughout the mouth and stimulated the appetite. Intake of the composition enriches living bodies with functional ingredients such as vitamins, minerals, and edible fibers inherent to Korean picked Chinese cabbage, and augments the active oxygen eliminating activity to the living bodies. The composition can be used to maintain and promote health, prevent aging and geriatric disease, promotes the treatment of incurable diseases, and prevent carcinogenesis. Thus, the composition can be used as a food product, cosmetic, pharmaceutical, or components or processing intermediates thereof.

### Example B-16

### Chewing Gum

Three parts by weight of a gum base was heated to be softened, then mixed with three parts by weight of crystalline cyclotetrasaccharide hydrate, three parts by weight of crystalline trehalose hydrate, one part by weight of a composition containing *aojiso* obtained by the method of Example B-9, and 0.01 parts by weight of a tea extract. The resulting mixture was admixed with adequate amounts of flavor and color, kneaded by a roll in a conventional manner, shaped, and packaged into a product having an active oxygen eliminating activity of about 120 units per gram product. The product is a chewing gum with satisfactory texture, flavor, and taste. The product containing cyclotetrasaccharide and trehalose as a saccharide is not easily utilized by dental caries inducing microorganisms, and it does not cause dental caries.

### Example B-17

### Dango (a Rice Paste)

Ten parts by weight of glutinous rice starch was mixed with 12 parts by weight of water, and the mixture was gelatinized by heating, then mixed with 0.5 part by weight of a composition containing mugwort obtained by the method of Example B-10. The mixture thus obtained was shaped and packaged in a conventional manner to produce a *dango* having an active oxygen eliminating activity of over 12 units per gram content. The product is a mugwort *- dango,* i.e., a rice paste with mugwort, having the inherent color tint, flavor, and taste to mugwort, and a satisfactory mouth feeling.

### Example B-18

### Nutritional Product

A composition was prepared from the following ingredients: 20 parts by weight of crystalline α-maltose, 1.1 parts by weight of glycine, 0.18 part by weight of sodium glutamate, 1.2 parts by weight of salt, one part by weight of citric acid, 0.4 part by weight of calcium lactate, 0.3 part by weight of a composition containing spinach obtained by the method of Example B-1, 0.01 part by weight of thiamine, and 0.01 part by weight of riboflavin. Twenty four grams of the composition were injected into small laminated aluminum bags and heat sealed to obtain a nutritional product for intubtaion having an active oxygen eliminating activity of over 12 units per gram composition. One bag of the nutritional product is dissolved in about 300-500 ml of water, and the solution can be administered to subjects orally or through the subjects' nasal cavities, stomachs, or intestines as a parenteral liquid nutritional supplement.

### Example B-19

### Cosmetic Cream

Two parts by weight of polyoxyethylene glycol monostearate, five parts by weight of glyceryl monostearate of self-emulsifying, 2.8 parts by weight of a composition containing aloe obtained by the method in Example B-12, 0.2 part by weight of α-glucosyl rutin, one part by weight of liquid petrolatum, 10 parts by weight of glyceryl trioctanate, and an adequate amount of an antiseptic were dissolved by heating in a conventional manner. The resulting solution was admixed with two parts by weight of L-lactic acid, five parts by weight of 1,3-butylene glycol, and 66 parts by weight of refined water, and the mixture was emulsified by a homogenizer and admixed with an adequate amount of a flavor under stirring to obtain a cosmetic cream having an active oxygen eliminating activity of about 46 units per gram product. The product can be used as a therapeutic or preventive agent for sunburned skin, as a skin beautifying agent, a skin whitening agent, and an agent for inhibiting aging of skin including chloasmas, freckles, pigmentation, and wrinkles.

### Example B-20

### Bath Salts

Bath salts having an active oxygen eliminating activity of about 220 units per gram product were prepared by mixing 26 parts by weight of refined water and adequate amounts of a coloring agent and flavoring agent with 21 parts by weight of sodium DL-lactate, eight parts by weight of sodium pyruvate, five parts by weight of a composition containing citron obtained by the method of Example B-7, one part by weight of α-glucosyl rutin, and 40 parts by weight of ethanol. The product can be suitable used as a skin beautifying agent and skin whitening agent by diluting 100-10,000 fold in hot water in a bathtub. Similarly as above, the product can be used by diluting it in water for a face wash or beauty wash.

### Example B-21

### Ointment

One part by weight of sodium acetate trihydrate and four parts by weight of calcium DL-lactate were mixed to homogeneity with 10 parts by weight of glycerin, and this mixture was added to a mixture consisting of 50 parts by weight of petrolatum, 10 parts by weight of vegetable wax, five parts by weight of lanolin, 14.5 parts by weight of sesame oil, six parts by weight of a composition containing *dokudami* obtained by the method of Example B-11, and 0.5 part by weight of peppermint oil. The mixture thus obtained was homogeneously mixed into an ointment having an active oxygen eliminating activity of about 54 units per gram. The product can be used as an antipyogenic agent, skin beautifying agent, skin whitening agent, and agent for promoting the treatment of skin traumas and burns.

As described above, the present invention provides a reduction inhibitory agent for active oxygen eliminating activity which comprises cyclotetrasaccharide as an effective ingredient, a method for inhibiting the reduction of active oxygen eliminating activity comprising incorporating either cyclotetrasaccharide or the reduction inhibitory agent into products to be treated, and a composition which contains plant edible substance and/or plant antioxidant in which the reduction of active oxygen eliminating activity is inhibited by the above method. Intake of the present composition easily nutritionally supplies living bodies and enriches living bodies with functional ingredients such as vitamins, minerals, and edible fibers of plant edible substances. Therefore, the present composition contributes greatly to maintain and promote health, prevent aging and geriatric diseases promote the treatment of incurable diseases, and inhibit carcinogenesis. Thus, the present invention provides a novel health resource as a fourth functional ingredient which can be used in processing and use of plant edible substances. The present invention provides a great contribution to a wide variety of fields, particularly the fields of food products, cosmetics, and pharmaceuticals.

## Claims

1. A method for inhibiting the reduction of active oxygen eliminating activity in a plant substance, which comprises a step of incorporating cyclotetrasaccharide represented by Chemical Formula 1 into said plant substance with active oxygen eliminating activity in an aqueous system.

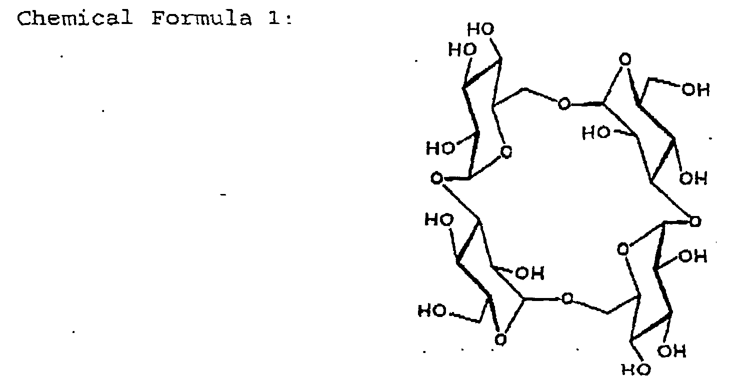

2. The method of claim 1, wherein said plant substance contains an antioxidant.

3. The method of claim 1, wherein said plant substance is in the form of a sliced disrupted, pulverized, dried, pickled, or extracted plant edible part.

4. The method of claim 2, wherein said plant antioxidant is a member selected from the group consisting of plant enzymes, pigments, polyphenols, and vitamins.

5. The method of any one of claims 1 to 4, wherein said cyclotetrasaccharide is incorporated into said plant substance in an amount of at least one percent (w/w) of said plant substance on a dry solid basis.

6. The method of any one of claims 1 to 5, which contains a sterilization step and/or a drying step.

7. The method of any one of claim 1 to 6, which further comprises a step of incorporating trehalose, pullulan, cyclodextrin, or a mixture thereof.

8. Use of cyclotetrasaccharide represented by Chemical Formula 1 for inhibiting the reduction of active oxygen eliminating activity in a plant substance.

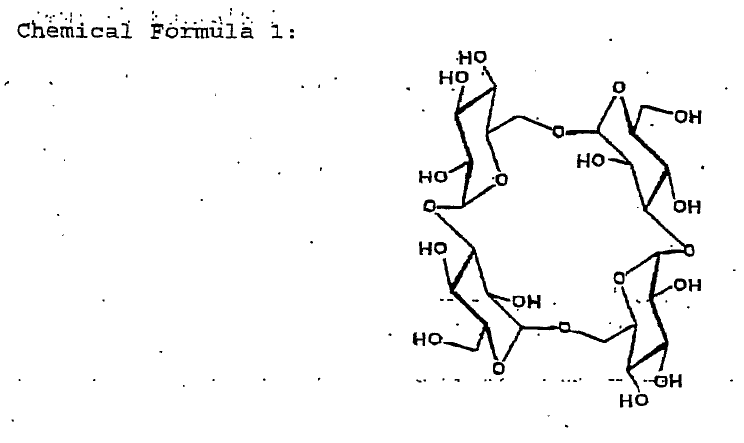

## Patentansprüche

1. Ein Verfahren zur Hemmung der Reduzierung der Aktivität zur Eliminierung von aktivem Sauerstoff in einer pflanzlichen Substanz, das einen Schritt zur Aufnahme des Cyclotetrasaccharids nach der chemischen Formel 1 in die pflanzliche Substanz mit einer Aktivität zur Eliminierung von aktivem Sauerstoff in einem wässrigen System enthält.

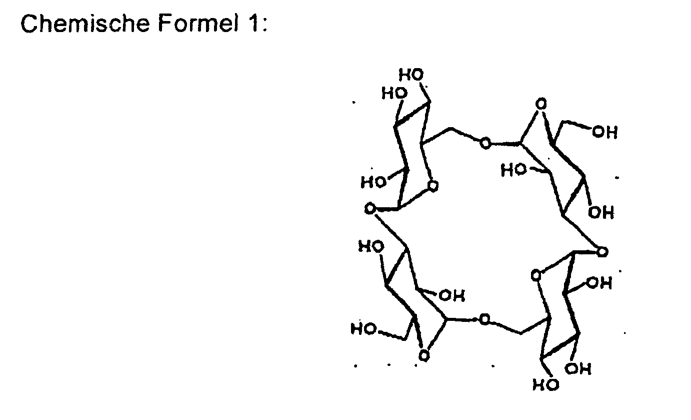

2. Das Verfahren nach Anspruch 1, wobei die pflanzliche Substanz ein Antioxidans enthält.

3. Das Verfahren nach Anspruch 1, wobei die pflanzliche Substanz in der Form eines geschnittenen, zerkleinerten, gepulverten, getrockneten, eingelegten oder extrahierten essbaren Pflanzenteils vorliegt.

4. Das Verfahren nach Anspruch 2, wobei das pflanzliche Antioxidans ausgewählt ist aus der Gruppe, die aus pflanzlichen Enzymen, Pigmenten, Polyphenolen und Vitaminen besteht.

5. Das Verfahren nach einem der Ansprüche 1 bis 4, wobei das Cyclotetrasaccharid in einer Menge von mindestens 1% (w/w) der pflanzlichen Substanz auf der Basis eines trockenen Feststoffs in die pflanzliche Substanz eingebracht wird.

6. Das Verfahren nach einem der Ansprüche 1 bis 5, das einen Sterilisationsschritt und/oder Trocknungsschritt enthält.

7. Das Verfahren nach einem der Ansprüche 1 bis 6, das ferner einen Schritt zur Aufnahme von Trehalose, Pullulan, Cyclodextrin oder einer Mischung davon enthält.

8. Verwendung des Cyclotetrasaccharids nach der chemischen Formel 1 zur Hemmung der Reduzierung der Aktivität zur Eliminierung von aktivem Sauerstoff in einer pflanzlichen Substanz.

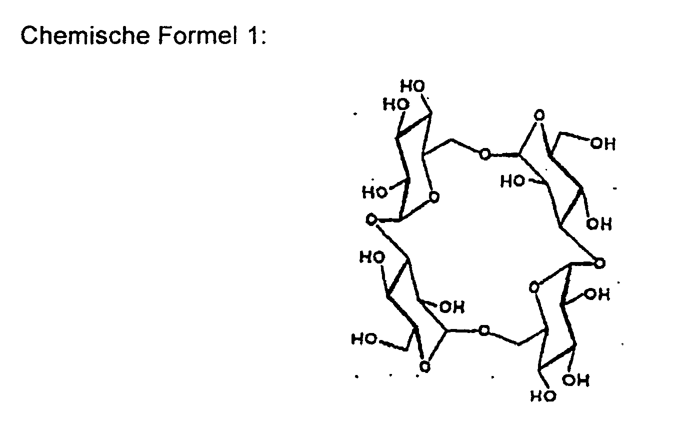

## Revendications

1. Procédé pour inhiber la réduction de l'activité d'élimination d'oxygène actif dans une substance végétale, qui comprend une étape d'incorporation d'un cyclotétrasaccharide représenté par la formule chimique 1 dans ladite substance végétale ayant une activité d'élimination d'oxygène actif dans un système aqueux.

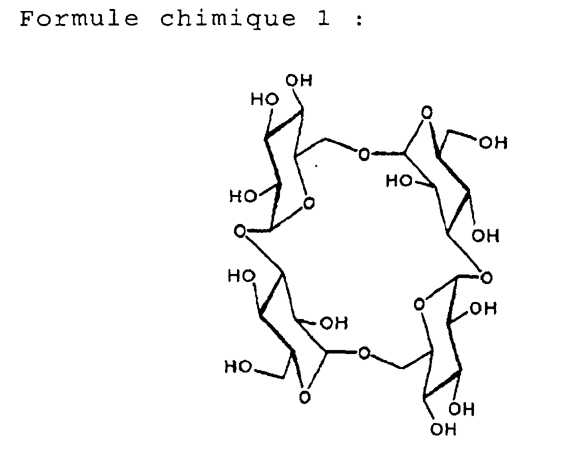

2. Procédé selon la revendication 1, dans lequel ladite substance végétale contient un antioxydant.

3. Procédé selon la revendication 1, dans lequel ladite substance végétale est sous la forme d'une partie comestible de la plante tranchée, dissociée, pulvérisée, séchée, marinée, ou extraite.

4. Procédé selon la revendication 2, dans lequel ledit antioxydant végétal est un élément choisi dans le groupe constitué par les enzymes, les pigments, les polyphénols, et les vitamines végétaux.

5. Procédé selon l'une quelconque des revendications 1 à 4, dans lequel ledit cyclotétrasaccharide est incorporé dans ladite substance végétale en une quantité d'au moins un pour-cent (p/p) de ladite substance végétale sur la base de l'extrait sec.

6. Procédé selon l'une quelconque des revendications 1 à 5, qui comprend une étape de stérilisation et/ou une étape de séchage.

7. Procédé selon l'une quelconque des revendications 1 à 6, qui comprend en outre une étape d'incorporation de tréhalose, de pullulane, de cyclodextrine, ou d'un de leurs mélanges.

8. Utilisation d'un cyclotétrasaccharide représenté par la formule chimique 1 pour inhiber la réduction de l'activité d'élimination d'oxygène actif dans une substance végétale.
